Europäisches Patentamt

European Patent Office

Office européen des brevets

(11) Publication number: **0 097 863**
**A2**

## (12) EUROPEAN PATENT APPLICATION

(21) Application number: 83105769.0

(22) Date of filing: 13.06.83

(51) Int. Cl.³: **A 61 M 1/03**
**A 61 M 5/00, B 01 D 13/00**
**A 61 L 2/02**

(30) Priority: 30.06.82 GB 8218841

(43) Date of publication of application:
11.01.84 Bulletin 84/2

(84) Designated Contracting States:
AT BE CH DE FR GB IT LI LU NL SE

(71) Applicant: Gambro Lundia AB
Box 10101
S-220 10 Lund(SE)

(72) Inventor: Shaldon, Stanley
Villa 86 Rue de Grezac
F-34100 Montpellier(FR)

(72) Inventor: Beau, Marie Claude
5, Rue Aquitaine
F-30000 Nimes(FR)

(74) Representative: Boberg, Nils Gunnar Erik
Gambro AB Patent Department Box 10101
S-220 10 Lund(SE)

(54) Mixing fluids by means of semipermeable membranes.

(57) Methods for mixing fluids in a filter member including a filtration membrane are disclosed. The methods include supplying a first fluid through an inlet to one side of the filtration membrane, supplying a second fluid through a second inlet to that same side of the filtration membrane, and withdrawing the mixture of those fluids from the other or filtrate side of the filtration membrane through a fluid outlet. The method has particular application in the medical field, such as in connection with the mixing of blood components and related fluids.

EP 0 097 863 A2

# MIXING FLUIDS BY MEANS OF SEMIPERMEABLE MEMBRANES

## FIELD OF THE INVENTION

The present invention relates to methods for mixing fluids. More particularly, the present invention relates to mixing fluids used in connection with various medical applications.

## BACKGROUND OF THE INVENTION

It is often desirable to mix two fluids, and to recover a purified product, particularly in the medical field. For example, during hemodialysis, a concentrate which is generally supplied by the manufacturer is mixed with a predetermined quantity of treated water which has been generated on site, to produce a dialysate fluid having a predetermined composition. The concentrate is normally about 2 to 3% by volume of the diluent, and may be mixed therewith in either batch or continuous form. Commercial equipment in the form of proportioning pumps, along with necessary control and monitoring circuits, may be employed for such continuous mixing processes. Sanitary procedures are of course employed, but it is not always feasible to guarantee sterility in the mixed solution, even when the two starting solutions are sterile. From time to time problems of pyrogenicity and septicity are thus encountered in clinical hemodialysis. Also the adequacy of the mixing of the concentrate and diluting fluid obtained is always a concern, and requires special equipment design features. Similarly, during the blood purification processes of hemofiltration and CAPD, large quantities of fluid containing specific quantities of electrolyte and other additives are required for infusion directly into the patient, and generally they must also be sterile and pyrogen-free. In order to insure such sterility, the electrolytes and other constituents are added at the time of manufacture, and the mixture can then be sterilized prior to shipment. Since several hundred gallons of fluid are required per patient per year, this procedure can become quite expensive. It can also involve a· considerable risk, in view of the potentially long time lag between any (unintentional) breach of sterility and final use, which will thus allow for the proliferation of bacteria therein.

As another example, in the case of hyperalimentation, amino acids and other constituents are mixed with water during manufacture, and then sterilized and shipped as a mixture. In this case as well, it might therefore ·prove advantageous to mix the injectable fluid on site from a concentrate.

## SUMMARY OF THE INVENTION

In accordance with the present invention, it has now been discovered that these and other objects can be attained by a method of mixing first and second fluids in a filter member including a filtration membrane having an inlet side and a filtrate side, first and second fluid inlets in fluid communication with the inlet side of the filtration membrane, and a fluid outlet in fluid communication with the filtrate side of the filtration membrane. The method includes supplying a first fluid to the inlet side of the filtration membrane through the first fluid inlet, supplying a second fluid to the inlet side of the filtration membrane through the second fluid inlet, and withdrawing the mixture of first and second fluids from the filtrate side of the filtration membrane through the fluid outlet. In a preferred embodiment, the first and second fluid inlets are located at opposite ends of the filter member.

In accordance with one embodiment of the method of the present invention, the filtration membrane includes a plurality of hollow membrane fibers, the inlet side of the filtration membrane comprising the intraluminal region within the hollow fibers and the filtrate side of the membrane comprising the extraluminal region outside of the hollow fibers. In accordance with a preferred embodiment, the hollow membrane fibers include first and second ends, the first fluid inlet being associated with the first end of the hollow fibers and the second fluid inlet being associated with the second end of the hollow fibers.

In accordance with another embodiment of the method of the present invention, the filtration membrane comprises an anisotropic capillary filter.

In accordance with another embodiment of the method of the present invention, the first fluid is water and the second fluid is a concentrate solution for addition to blood, whereby the mixture of first and second fluids comprises a sterile and pyrogen-free mixture. In accordance with an embodiment of the method of the present invention, the filtration membrane can comprise a hyperfiltration membrane, an ultrafiltration membrane, or a microporous membrane, depending upon the ultimate use thereof.

In accordance with a preferred embodiment of the method of the present invention, in which hollow membrane fibers are utilized, they preferably will have a diameter of less than about 0.5 mm. Preferably, at least about 5,000 such hollow membrane fibers will be employed.

In accordance with a preferred embodiment of the method of the present invention, the method includes monitoring the mixture ratio of the mixture withdrawn through the fluid outlet, and preferably this is done by means of a conductivity cell. In a preferred embodiment, the method includes pumping the first fluid to the first fluid inlet at a first predetermined rate, and controlling that first predetermined rate on the basis of the monitored mixture ratio.

The method of this invention has thus been found to provide high quality mixing of fluids. It has thus been found that in addition to the fluids being mixed, the resultant mixture can now also be sterile. As a consequence, the present invention becomes a particular advantage for use in medical applications, such as infusate preparation for hemofiltration.

Certain classes of microporous membranes will produce a sterile filtrate from a non-sterile feed, and certain classes of ultrafiltration membranes will yield a sterile and non-pyrogenic filtrate from feed streams which are not sterile and which are non-pyrogenic. Such membranes are available in the form of flat sheets, as tubes, and as hollow fibers.

In a preferred embodiment of this invention, however, a hollow fiber membrane filter is used by applying each of the fluids to a respective end of the intraluminal region of a plurality of hollow fiber membranes, and removing the mixture as a filtrate from the extraluminal region of the membranes.

Although maximum results may thus be provided by using hollow fiber membrane filters, it is also apparent that other forms of membrane filters may also be employed, built up for example in the form of conventional flat membrane dialyzers or dialyzers including larger membrane tubes.

Hollow fiber membranes are available as filtration devices which have a geometry similar to that of a shell and tube heat exchanger. They thus contain an inlet manifold, an inlet header in which the open ends of the fibers are exposed to the inlet manifold, the intraluminal region of the fibers, an outlet header, and an outlet manifold similar to the inlet counterparts. The inlet and outlet headers are composed of thermosetting potting, which also serves to isolate the manifolds and luminal region from the filtrate chamber (i.e., extraluminal volume). The filtrate may then be recovered from the extraluminal volume by means of a filtrate port or outlet. The hollow fibers may be composed of several types of membranes which are well known to the art, and which have the following general properties, including:

Hyperfiltration membranes — retentive to salt;

Ultrafiltration membranes — pass electrolytes, sugars and low MW substances, but retain proteins, and unlysed pyrogens obtained from gram negative bacteria, and viruses; and

Microporous membranes — pass proteins and some pyrogens, but retain bacteria, some viruses, etc.

A special advantage which can now be realized by supplying the fluids which are to be mixed to two opposite inlets of a membrane filter and removing the mixed fluids from an outlet for the filtrate, is that you can thus avoid the presence of any dead end in the filter so utilized. Instead, the two fluids meet in a "living" mixing zone.

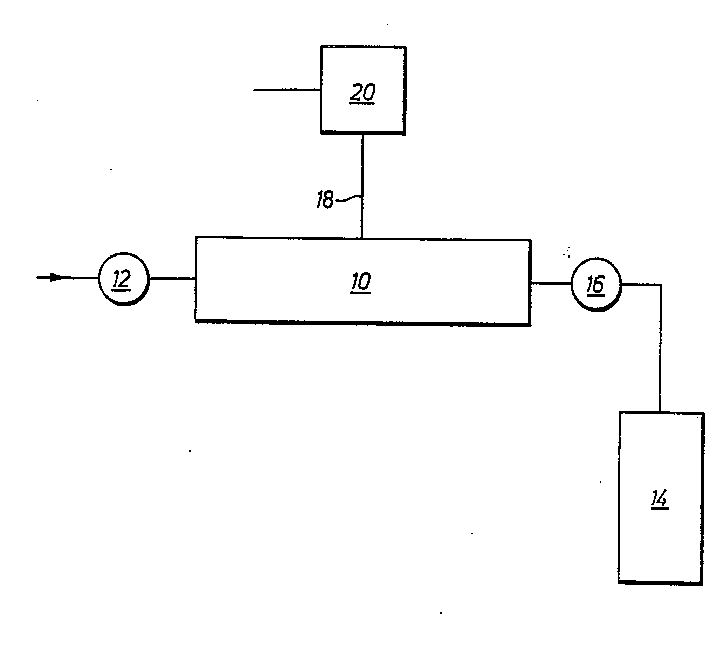

## BRIEF DESCRIPTION OF THE FIGURE

One embodiment of the method of the present invention will now be described by way of example only, and with reference to the accompanying figure, wherein:

The figure is a schematic representation of a method of the present invention for mixing and simultaneously sterilizing water and a concentrate in order to provide a sterile mixture, such as an infusate.

## DETAILED DESCRIPTION

While the present invention will thus be described in terms of a particular application thereof, it will be appreciated that the invention is applicable to the production of many forms of intravenous fluids.

The particular preferred form of the present invention which is described below is predicated upon the fact that an anisotropic capillary filter can be used to mix fluids so as to produce a high quality mixture at its filtered output. Anisotropic capillary filters are conventionally used in hemofiltration processes in which blood from a patient is passed through the filter, and products such as water are drawn off through the filtered output and evacuated to waste. The filtered blood is mixed with a suitable infusate prior to recirculation to the patient in order to provide replacement constituents for those drawn off during filtration, while avoiding the undesired constituents.

Preferably, the respective fluids are supplied through the opposing manifolds of a hollow fiber ultrafilter. The fluids mix in the intraluminal capillary region, and the mixture then passes through the filter and is recovered in the filtrate compartment. Mixing is facilitated by the small diameter of the fibers, and by the confluence of two oppositely directed streams. Thus, the single process provides for both the intimate admixture of two streams and the recovery of a purified product.

In the case of hemodialysis, hemofiltration, and CAPD, the membrane would be of the ultrafiltration class specified above, and with a molecular weight (MW) cutoff of from about 500 to 50,000 MW. The purified product would be sterile and pyrogen free.

This invention is also adaptable to a wide 0097863 of flow conditions and geometric configurations. Typically, however, the fluid is passed through the mixer-purifier at flow rates equivalent to those which are demanded by the particular process, e.g., from about 60 to 200 cc/min for hemofiltration, and from about 300 to 600 cc/min for hemodialysis. Preferably, the fiber diameter should be less than about 0.5 mm, so as to assure good mixing. The device may have a filter area ranging from about 100 cm$^2$ to several square meters, again depending upon the particular end application.

Referring next to the figure, the fluids which are to be mixed are supplied to respective ends of an anisotropic capillary filter 10. In the embodiment shown, pretreated water is thus supplied by a pump 12 and a concentrate is supplied from a reservoir 14 by a pump 16, to the filter 10.

The filter 10 is in the form of a commercially available unit comprising a hollow glass or plastic tube packed with approximately 5,000 capillary elements. The water and concentrate are each supplied to a respective header, each of which is associated with a respective end of the tube containing the capillaries. Under the action of pumps 12 and 16, the water and concentrate thus flow through the capillaries and pass through the walls of the capillaries. It has been found that a high quality mixture is provided at the output 18 of the filter 10, and this appears to be at least partly due to the capillary mixing of the fluids which occurs as the fluids pass across the walls of the capillaries.

It is known that in passing through the walls of the capillaries the fluids are cleansed of pyrogens and bacteria. It is therefore possible to introduce water into the filter 10, via pump 12, which is not sterile, and in mixing the water with the concentrate in the filter 10, a sterile mixture is obtained at the outlet 18 of the filter 10.

A conductivity cell 20 is attached to the output 18 of the filter 10. Conductivity cell 20 monitors the relative proportion of concentrate in the output, and since the conductivity cell 20 can be coupled with pump 16, it is possible to produce a mixture
at the output 18 which has an automatically controlled percentage
of concentrate. The pump 16 may comprise a variable photo-ohmic
speed control, which can thus be more readily coupled with the
conductivity cell 20.

The output from conductivity cell 20 could be supplied
to a sterile, closed polyvinylchloride sack, or directly to a
monitoring machine, for use, for example, in hemodialysis or
hemofiltration processes.

The water supplied via the pump 12 is pretreated, and
may be continuously supplied from a reverse osmosis apparatus.
A typical ratio of fluids in the final mix is about 34 parts of
water to 1 part of concentrate.

The above description has been given in relation to the
preparation of an infusate mixture, but it is to be understood
that the method of the present invention is similarly suitable
for preparation of other mixtures.

It will be understood that the embodiment described
herein is merely exemplary and that a person skilled in the art
may make many variations and modifications without departing from
the spirit and scope of the invention. All such modifications and
variations are intended to be included within the scope of the
invention as defined in the appended claims.

WHAT IS CLAIMED IS:

1. A method of mixing first and second fluids in a filter member including a filtration membrane having an inlet side and a filtrate side, first and second fluid inlets in fluid communication with said inlet side of said filtration membrane, and a fluid outlet in fluid communication with said filtrate side of said filtration membrane, said method comprising supplying said first fluid to said inlet side of said filtration membrane through said first fluid inlet, supplying said second fluid to said inlet side of said filtration membrane through said second fluid inlet, and withdrawing said mixture of said first and second fluids from said filtrate side of said filtration membrane through said fluid outlet.

2. The method of Claim 1 wherein said first and second fluid inlets are located at opposite ends of said filter member.

3. The method of Claim 1 wherein said filtration membrane comprises a plurality of hollow fibers, said inlet side of said filtration membrane comprising the intraluminal region within said hollow fibers and said filtrate side of said filtration membrane comprising the extraluminal region outside said hollow fibers.

4. The method of Claim 3 wherein said hollow fibers include first and second ends, said first fluid inlet being associated with said first end of said hollow fibers and said second fluid inlet being associated with said second end of said hollow fibers.

5. The method of Claim 1 wherein said filtration membrane comprises an anisotropic capillary filter.

6. The method of Claim 1 wherein said first fluid comprises water and said second fluid comprises a concentrate solution for addition to blood, whereby said mixture of said first and second fluid comprises a sterile and pyrogen-free mixture.

7. The method of Claim 1 wherein said filtration membrane comprises a hyperfiltration membrane.

8. The method of Claim 1 wherein said filtration membrane comprises an ultrafiltration membrane.

9. The method of Claim 1 wherein said filtration membrane comprises a microporous membrane.

10. The method of Claim 3 wherein said hollow fibers have a diameter of less than about 0.5 mm.

11. The method of Claim 3 wherein said plurality of hollow fibers comprise at least about 5,000 hollow fibers.

12. The method of Claim 1 including monitoring the mixture ratio of said mixture withdrawn through said fluid outlet.

13. The method of Claim 12 wherein said monitoring of said mixture ratio is carried out by means of a conductivity cell.

14. The method of Claim 12 including pumping said first fluid to said first fluid inlet at a predetermined rate and controlling said predetermined rate on the basis of said mixture ratio.